(19) Europäisches Patentamt
European Patent Office
Office européen des brevets

(11) Veröffentlichungsnummer: **0 073 505**
**B1**

# (12) EUROPÄISCHE PATENTSCHRIFT

(45) Veröffentlichungstag der Patentschrift: **27.11.85**

(51) Int. Cl.⁴: **C 07 D 265/36**, C 07 D 263/58, A 61 K 31/42, A 61 K 31/535

(21) Anmeldenummer: **82107919.1**

(22) Anmeldetag: **28.08.82**

## (54) Neue Benzo-Heterocyclen.

(30) Priorität: **01.09.81 DE 3134590**

(43) Veröffentlichungstag der Anmeldung: **09.03.83 Patentblatt 83/10**

(45) Bekanntmachung des Hinweises auf die Patenterteilung: **27.11.85 Patentblatt 85/48**

(84) Benannte Vertragsstaaten: **AT BE CH DE FR IT LI LU NL SE**

(56) Entgegenhaltungen:
**DE - A - 2 005 134**
**DE - A - 2 429 253**

**Chemical Abstracts Band 90, Nr. 7, 12 Februar 1979, Columbus, Ohio, USA L.J. RAVIN et al. "Degradative behavior of a new bronchodilator carbuterol, in aqueous solution" Seite 544, Spalte 2, Abstract Nr. 54156g**

**Die Akte enthält technische Angaben, die nach dem Eingang der Anmeldung eingereicht wurden und die nicht in dieser Patentschrift enthalten sind.**

(73) Patentinhaber: **BOEHRINGER INGELHEIM KG, D-6507 Ingelheim am Rhein (DE)**

(72) Erfinder: **Schromm, Kurt, Dr., In der Dörrwiese 35, D-6507 Ingelheim/Rhein (DE)**
Erfinder: **Mentrup, Anton, Dr., Steinernstrasse 25, D-6503 Mainz-Kastel (DE)**
Erfinder: **Renth, Ernst-Otto, Dr., Frankenstrasse 11, D-6507 Ingelheim/Rhein (DE)**
Erfinder: **Fügner, Armin, Dr., Im Hippel 31, D-6535 Gau-Algesheim (DE)**

**EP 0 073 505 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Benzo-Heterocyclen, die als Wirkstoffe für Arzneimittel verwendbar sind.

Die neuen Verbindungen entsprechen der allgemeinen Formel

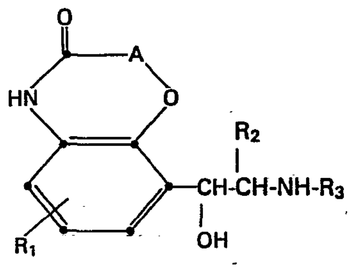

(I),

in der

A eine Einfachbindung, die Gruppe $=C\langle^{R_5}_{R_4}$ oder die Gruppe $-CH_2-CH_2$,

$R_1$ OH, O-($C_1$-$C_6$-Alkanoyl), O-Benzoyl, O-p-Methylbenzoyl oder Chlor,

$R_2$ Wasserstoff, Methyl oder Ethyl,

$R_3$ 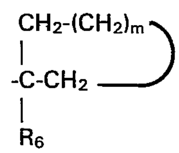 oder 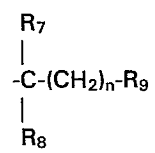

(II) (III)

m 2, 3 oder 4,

n 1, 2 oder 3,

$R_4$ Wasserstoff, $C_1$-$C_4$-Alkyl

$R_5$ Wasserstoff, $C_1$-$C_4$-Alkyl und ausserdem, falls $R_4$ Wasserstoff ist, Phenyl,

$R_6$ Wasserstoff oder Methyl,

$R_7$ Wasserstoff oder Methyl,

$R_8$ Wasserstoff oder Methyl,

$R_9$ Wasserstoff, Ar, OAr, NH-CO-Ar

Ar 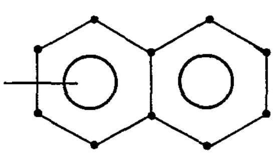 , 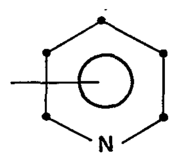 oder

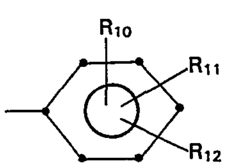

(IV)

$R_{10}$, $R_{11}$, $R_{12}$ (die gleich oder verschieden sein können), Wasserstoff, Hydroxy, Methyl, Methoxy, Halogen, Methylendioxy, NH-$R_{13}$, $CONH_2$,

$R_{13}$ Wasserstoff, $C_1$-$C_6$-Alkanoyl oder Benzoyl oder $C_1$-$C_4$-Alkylsulfonyl bedeutet; sie können in Form von Racematen, Enantiomeren und gegebenenfalls diastereomeren Antipodenpaaren als freie Basen oder als Säureadditionssalze vorliegen.

Im Sinne der Erfindung bedeutet «niederes Alkyl» einen Alkylrest mit 1 bis 4 Kohlenstoffatomen, «Halogen» Fluor, Chlor, Brom und Jod, bevorzugt Fluor und Chlor. Die O-($C_1$-$C_6$-Alkanoyl)-Gruppe kann geradkettig oder verzweigt sein.

In einer bevorzugten Ausführungsform der Erfindung steht

A für eine Einfachbindung, $=CH_2$, $=CH(CH_3)$, $=C(CH_3)_2$ oder $=CH(C_2H_5)$,

$R_1$ für OH oder O-$C_1$-$C_6$-Alkanoyl oder O-Benzoyl in m- oder p-Stellung zur Seitenkette,

$R_2$ für H, $CH_3$ oder $C_2H_5$,

$R_3$ für die oben angegebenen Reste II und III,

m für 2 oder 3,

n für 1, 2 oder 3,

$R_6$, $R_7$, $R_8$ für Wasserstoff oder Methyl,

$R_9$ für H, Ar, NH-CO-Ar,

Ar für den Rest der Formel IV, 2-Pyridyl oder 4-Pyridyl,

$R_{10}$ für H, OH, $CH_3$, gemeinsam mit $R_{11}$ auch Methylendioxy,

$R_{11}$ für H, OH, $CH_3$, gemeinsam mit $R_{10}$ auch Methylendioxy,

$R_{12}$ für H,

$R_{13}$ für Acetyl oder Methansulfonyl.

Besonders hervorzuheben sind die Verbindungen, in denen

A für $=C(CH_3)_2$ oder $-CH_2-$,

$R_1$ für OH in p- oder m-Stellung zur Seitenkette,

$R_2$ für H, $CH_3$ oder $C_2H_5$,

$R_3$ für Isopropyl, tert.-Butyl, Cyclopentyl, 1-Methylcyclopentyl oder den Rest der Formel III,

n für 1 oder 2,

$R_7$, $R_8$ für Wasserstoff oder Methyl,

$R_9$ für Phenyl, 4-Hydroxyphenyl, 2-Pyridyl, 4-Pyridyl, 2-Hydroxyphenyl,

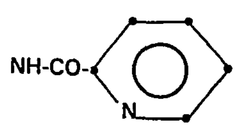 , 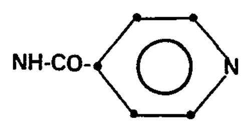 ,

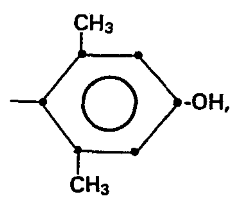 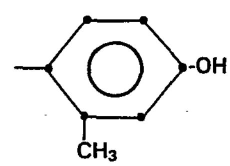

steht.

In der DE-A1 2 429 253 sind Phenylalkanolamine bekannt, die eine gewisse strukturelle Verwandtschaft zu den erfindungsgemässen Verbindungen aufweisen. Während die bekannten Verbindungen jedoch vor allem zur Behandlung von Bluthochdruck und Kreislaufschäden vorgeschlagen wurden, zeigen die neuen Verbindungen überraschenderweise insbesondere broncholytische, spasmolytische und antiallergische Eigenschaften.

Aus Chemical Abstracts, Band 90, Nr. 7, Abstr. Nr. 54 156 g ist ein 1-Benzoxazolidinonyl-2-t-butyl-aminoethanol bekannt, das broncholytische Eigenschaften aufweist. Da das Sauerstoffatom des ankondensierten heterocyclischen Rings, der zudem ein Fünfring ist, sich in 4-Position zur Seitenkette befindet, steht die bekannte Verbindung den erfindungsgemässen Verbindungen so fern, dass die

broncholytische Wirkung der neuen Verbindungen nicht nahe lag.

Die neuen Verbindungen werden nach an sich bekannten Methoden hergestellt.

1. Reduktion von Verbindungen der Formel

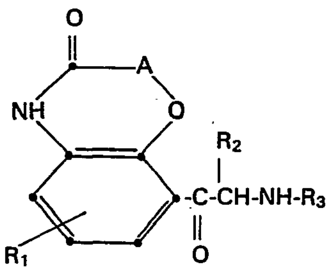

(V),

in der A, $R_1$, $R_2$ und $R_3$ die obige Bedeutung haben, wobei jedoch phenolische OH-Gruppen auch durch eine hydrogenolytisch abspaltbare Gruppe geschützt vorliegen können.

Die Reduktion wird in einem unter den Umsetzungsbedingungen ausreichend stabilen Lösungsmittel, z.B. in einem niederen Alkohol wie Ethanol, durchgeführt. Als Reduktionsmittel dienen Wasserstoff und Hydrierungskatalysatoren (wie Palladium, Platin, Raney-Nickel) oder Hydride (wie Natriumborhydrid oder Diboran). Durch geeignete Wahl des Reduktionsmittels (katalytische Reduktion bzw. Reduktion mit Hydriden) kann die Bildung vorwiegend der erythro- bzw. threo-Form erreicht werden.

Eventuell an der zentralen Aminogruppe oder an einer phenolischen Hydroxylgruppe vorhandene hydrogenolytisch abspaltbare Schutzgruppen, beispielsweise Benzyl oder substituiertes Benzyl, werden während oder nach der Reduktion auf übliche Weise entfernt.

Die neuen, als Ausgangsstoffe dienenden Verbindungen der Formel V können nach an sich bekannten Methoden erhalten werden, etwa wie in dem folgenden Reaktionsschema dargestellt ist:

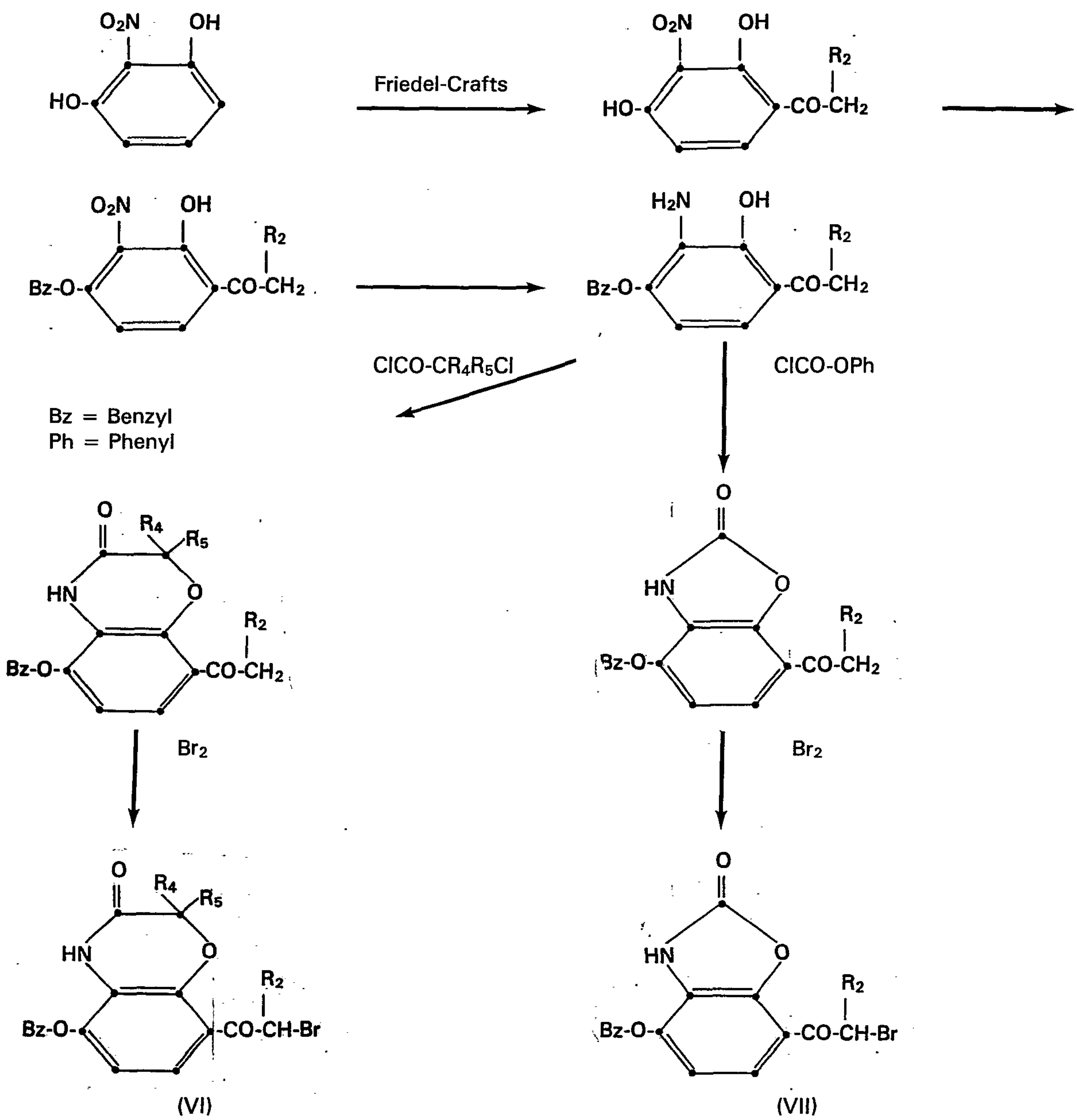


(VI) (VII)

Entsprechende Bromketone mit der (geschützten) OH-Gruppe in m-Stellung zur Seitenkette können wie folgt erhalten werden

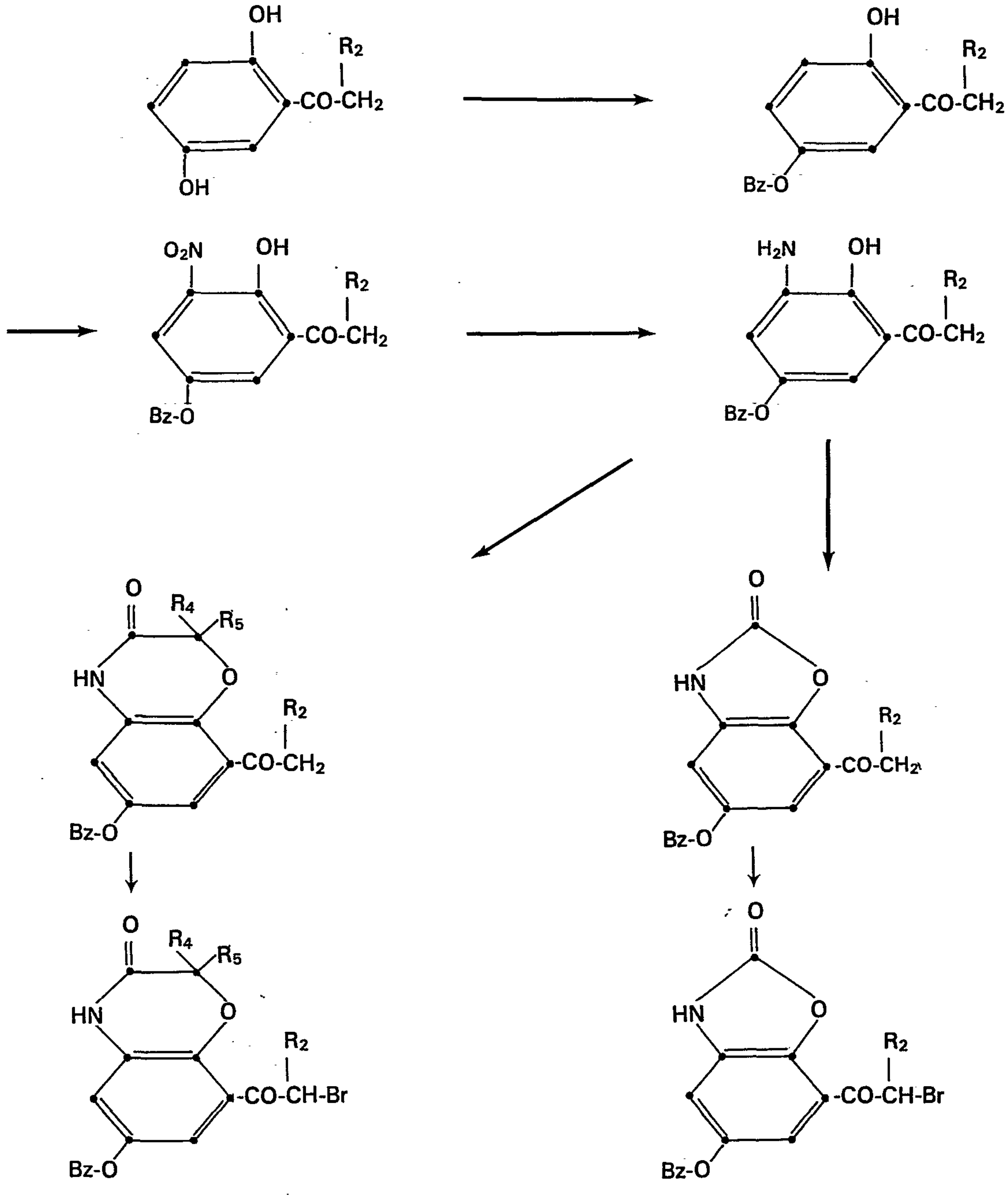

(VIII) (IX)

Die so oder nach anderen üblichen Methoden erhaltenen Bromketone der Formel

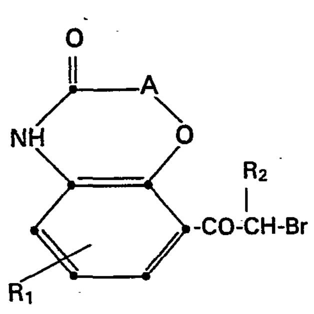

(X)

in der A, $R_1$ und $R_2$ die oben genannte Bedeutung haben, wobei jedoch phenolische OH-Gruppen durch hydrogenolytisch abspaltbare Gruppen, etwa Benzyl, geschützt vorliegen können, können mit Aminen der Formel

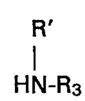

(XI),

worin $R_3$ die obige Bedeutung hat und R' Wasserstoff oder einen hydrogenolytisch abspaltbaren Rest, etwa Benzyl oder substituiertes Benzyl bedeutet, zu Verbindungen der Formel

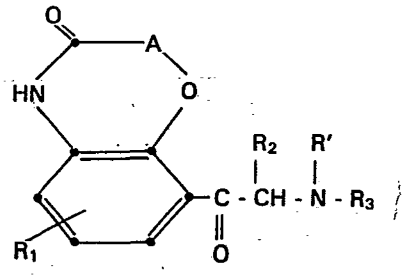

(Va)

worin A, $R_1$, $R_2$, $R_3$ und R' die oben genannten Bedeutungen haben, eingesetzt werden. Die Umsetzung erfolgt in geeigneten Lösungsmitteln, etwa Acetonitril, Essigester, in Gegenwart eines säureabfangenden Mittels, z.B. Natriumcarbonat oder Aminüberschuss. In dem Reaktionsprodukt vorhandene Schutzgruppen können anschliessend oder bei der weiteren Umsetzung bzw. danach entfernt werden.

2. Umsetzung von Phenylglyoxalen oder Halbacetalen der Formel

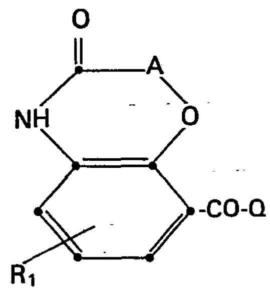

(XII),

in der $R_1$ und A die obige Bedeutung haben und Q für -CHO oder

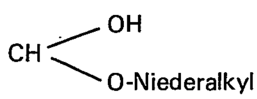

steht, mit Aminen der Formel

$H_2N-R_3$ (XIII),

in der $R_3$ die obige Bedeutung hat, unter den Bedingungen der reduktiven Aminierung.

Statt der Komponenten XII und XIII können auch die ggf. als Zwischenprodukt auftretenden Schiffschen Basen der Formel

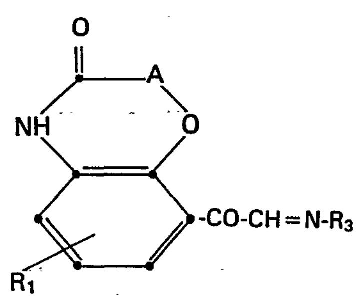

(XIV),

in der A, $R_1$ und $R_2$ die obige Bedeutung haben, einer Reduktion unterworfen werden.

Als Reduktionsmittel werden komplexe Hydride, vorzugsweise Natriumborhydrid, oder Wasserstoff und Hydrierungskatalysatoren wie Platin, Palladium, Nickel verwendet.

In den Ausgangsstoffen enthaltene phenolische Hydroxygruppen können durch übliche hydrogenolytisch abspaltbare Gruppen geschützt sein. Diese Schutzgruppen können während oder nach der Reduktion in üblicher Weise hydrogenolytisch entfernt werden.

Man erhält Endprodukte mit $R_2$ gleich Wasserstoff.

Die als Ausgangsstoffe dienenden Verbindungen der Formel XII können aus Acetophenonderivaten der Formel

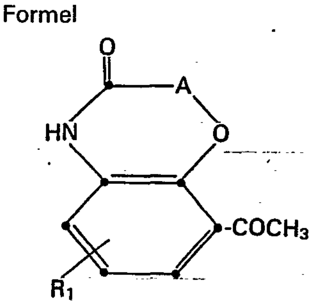

(XV),

in der $R_1$ und A die obige Bedeutung haben, durch Oxidation mit Selendioxid in wässrigem Dioxan erhalten werden. Je nach der Kristallisation aus Wasser oder niederen Alkoholen erhält man Glyoxale oder Halbacetale.

Die Amine der Formel XIII sind bekannt oder nach üblichen Methoden leicht zu gewinnen.

3. Entfernung von hydrogenolytisch abspaltbaren Schutzgruppen von Verbindungen der Formel

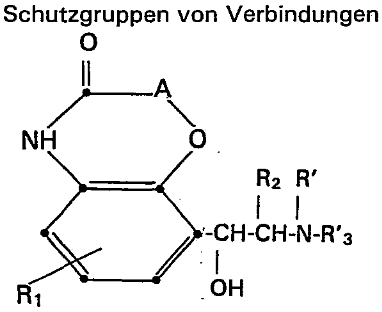

(XVI),

die durch Reduktion aus Verbindungen der Formel V bzw. Va, wie unter 1. beschrieben, zu erhalten sind, in der A und $R_2$ die obige Bedeutung haben und $R'_1$ für $R_1$ oder eine durch eine hydrogenolytisch abspaltbare Schutzgruppe geschützte OH-Gruppe, $R'_3$ für $R_3$ steht, wobei jedoch eine in $R_3$ etwa enthaltene OH-Gruppe durch eine hydrogenolytisch abspaltbare Schutzgruppe geschützt sein kann, und R' für Wasserstoff oder eine hydrogenolytisch abspaltbare Schutzgruppe steht. Als hydrogenolytisch abspaltbare Schutzgruppen kommen vor allem Benzyl und substituiertes Benzyl in Betracht.

Gewünschtenfalls werden die nach 1. bis 3. erhaltenen Verbindungen nach üblichen Methoden in die Enantiomeren, ggf. auch in die diastereomeren Antipodenpaare aufgetrennt, zunächst erhaltene Basen in Säureadditionssalze, zunächst erhaltene Säureadditionssalze in Basen oder Salze mit anderen Säuren überführt.

Die erfindungsgemässen Verbindungen sind als Arzneistoffe verwendbar. Sie haben u.a. broncholytische, spasmolytische und antiallergische Wirkung und erhöhen die Ciliartätigkeit und verringern entzündlich-exsudative Reaktionen. Sie sind daher für alle Formen von Asthma und Bronchitis, bei Urticaria, Konjunktivitis, Heufieber und Erkältungskrankheiten anwendbar. Sie sind ferner Relaxantien der Uterusmuskulatur, die geeignet sind, Beschwerden vor der Geburt zu verhindern. Die Verbindungen können auch zur Behandlung von cardiovaskulären Störungen, z.B. von Bluthochdruck, peripheren Gefässkrankheiten und Arrhythmien dienen. Als weitere Wirkungen werden Inhibitoren der Magensekretion und im ZNS antidepressive Effekte beobachtet.

Die therapeutische und prophylaktische Dosis ist abhängig von der Beschaffenheit und Ernsthaftigkeit des Krankheitszustandes und von der Verabreichungsart.

Für einen Erwachsenen sind pro die für folgende Indikationen folgende Dosierungen angezeigt.

Als Broncholytika oral 0,05 - 5 mg, inhalativ 0,01 - 1,0 mg und subcutan 0,02 - 0,5 mg zu nehmen.

Als Uterusmittel werden oral 10 - 50 mg oder als Infusionslösung Ampullen (10 ml), die 0,1 - 1 mg enthalten, angewandt.

Für die Gefässerweiterung werden oral 20 - 100 mg Ampullen für die i.m.-Injektion mit 20 - 40 mg verwendet. Die Blutdrucksenker sind oral mit 200 mg bis 1,8 g zu dosieren.

Die pharmazeutischen Zubereitungen enthalten Wirkstoffe, pharmazeutisch akzeptable Trägersubstanzen und gegebenenfalls andere therapeutische Bestandteile.

So sind die Broncholytika kombinierbar mit Theophyllinen, Parasympatholytika (z.B. Ipratropiumbromid), Sekretolytika (z.B. Bromhexin), muskulotropen Spasmolytika (z.B. Papaverin), Corticosteroiden und Antiallergika. Bei den Uterusrelaxantien sind Kombinationen mit Corticoiden möglich.

Für die orale Verabreichung eignen sich Kapseln, Tabletten, Lösungen und Suspensionen. Bei der pulmonalen Gabe werden vorzugsweise trockene Pulver mit Teilchendurchmesser von 0,5 - 7 μ mittels Treibgasen in den Bronchialbereich gebracht. Die parenterale Anwendung erfolgt zweckmässigerweise in Form steriler isotonischer wässeriger Lösungen. Für die lokale Anwendung werden Lotionen, Crems, Salben, Emulsionen und Sprays genommen.

Die erfindungsgemässen Verbindungen können auch angewandt werden, um die Wachstumsrate von fleischproduzierenden Tieren, z.B. Schweinen, Rindvieh, Schafen, Hühnern, Gänsen, zu vergrössern. Die Ausnutzung des Futters wird erheblich verbessert, ausserdem hat das Fleisch eine höhere Qualität und einen geringeren Fettgehalt als ohne die Anwendung der neuen Verbindungen.

*Formulierungsbeispiele:*

*Tabletten*

Zusammensetzung einer Tablette

| | |
|---|---|
| Wirkstoff gemäss der Erfindung | 20 mg |
| kolloidale Kieselsäure | 10 mg |
| Milchzucker | 118 mg |
| Kartoffelstärke | 60 mg |
| Polyvinylpyrrolidon | 6 mg |
| Na-Celluloseglykolat | 4 mg |
| Magnesiumstearat | 2 mg |
| | 220 mg |

*Ampullen*

Zusammensetzung der Lösung pro Ampulle

| | |
|---|---|
| Wirkstoff gemäss der Erfindung | 10 mg |
| Sorbit | 40 mg |
| destill. Wasser ad | 10 ml |

*Suppositorien*

Zusammensetzung pro Suppositorium

| | |
|---|---|
| Wirkstoff gemäss der Erfindung | 100 mg |
| Suppositorienmasse (Kakaobutter) | 1600 mg |
| | 1700 mg |

*Inhalationspulver*

Pro Hartgelatine-Steckkapsel werden 0,5 mg Wirkstoff gemäss der Erfindung und 19,5 mg Lactose mit einem Teilchendurchmesser zwischen 0,5 und 7 μm eingefüllt.

Für die pharmakologischen Prüfungen werden die üblichen Testmethoden und Versuchstiere oder Organe verwendet. Pharmakologisch sind die erfindungsgemässen Verbindungen gegenüber Handelsprodukten der gleichen Indikation zum Teil sehr deutlich unterschieden. Neben einer guten Wirkungsdauer zeigt sich z.B. eine besonders deutliche Selektivität im Verhältnis Broncholyse zu Herzfrequenzsteigerung. So ist z.B. für die Verbindung nach Beispiel 1 am Meerschweinchen die $ED_{50}$ i.v. [μg/kg] der Herzfrequenzsteigerung mehr als zehnmal so gross wie die $ED_{50}$ i.v. [μg/kg] der Broncholyse, die nur 0,045 μg/kg beträgt. Günstig sind im allgemeinen auch die Resorptionsverhältnisse. So ist der Resorptionsquotient

$$\frac{ED_{50}\ p.o.}{ED_{50}\ i.v.}$$

z.B. für die Verbindung 7 der Tabelle 3 nur 1,1, so dass die orale Wirkung praktisch ebenso gross ist wie die intravenöse. Die $LD_{50}$-Werte liegen, z.B. an der Maus, so weit über der therapeutischen Dosis, dass eine günstige therapeutische Breite gegeben ist.

Die nachstehenden Beispiele sollen die erfindungsgemässen Verfahren näher erläutern, da die Reaktionsbedingungen mit ähnlichem Ergebnis erheblich variiert werden können.

Je nach dem Lösungsmittel, aus dem die nachstehend erwähnten Substanzen kristallisiert werden, enthalten sie zum Teil noch definierte Mengen des Lösungsmittels im Kristall gebunden. Die angegebenen Schmelzpunkte sind unkorrigiert.

*Zu Verfahren 1*

*Beispiel 1*

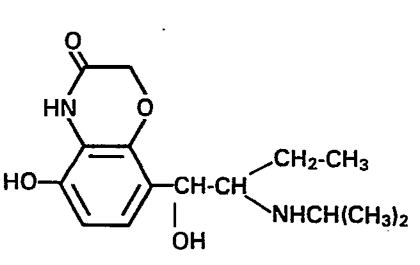

$\times HCl \times H_2O$

16,1 g 5'-Benzyloxy-8'-(1-oxo-2-brom-butyl)-2H-1,4-benzoxazin-3-(4H)-on und 7,5 g Isopropylamin werden in 100 ml Acetonitril 4 Stunden bei 60°C gerührt. Nach Ansäuern mit konz. Salzsäure und Zugabe zum 100 ml Wasser kristallisiert das 5'-Benzyloxy-8'-(1-oxo-2-isopropylamino-butyl)-2H-1,4-benzoxazin-3-(4H)-on hydrochlorid (Fp. 229 - 232°C) aus.

6 g dieser Verbindungen werden in Methanol unter Zusatz von Palladium-Kohle als Katalysator zu 5'-Hydroxy-8'-(1-oxo-2-isopropylamino-butyl)-2H-1,4-benzoxazin-3-(4H)-on hydrochlorid dihydrat (Fp. 242 - 245°C) entbenzyliert.

Durch Hydrierung von 3,3 g dieser Verbindung in Methanol mit Platin als Katalysator erhält man 3 g erythro-5'-Hydroxy-8'-(1-hydroxy-2-isopropylamino-butyl)-2H-1,4-benzoxazin-3-(4H)-on-hydrochlorid-hydrat (Ausbeute 90% d. Th.), welches bei 208 - 210°C schmilzt.

*Beispiel 1 a*

Verfahren 3

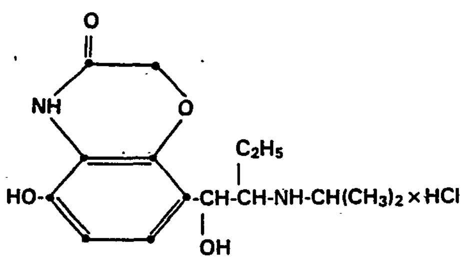

(threo)

32,4 g 5'-Benzyloxy-8'-(1-oxo-2-brom-butyl)-2H-1,4-benzoxazin-3-(4H)-on und 72 g Benzylisopropylamin werden 15 Stunden bei 100°C gerührt. Nach Zusatz von Wasser wird das ausgefallene Öl mit Äther aufgenommen und mit Petroläther verdünnt; es erfolgt Kristallisation von 5'-Benzyloxy-8'-(1-oxo-2-benzylisopropylamino-butyl)-2H-1,4-benzoxazin-3-(4H)-on.

11,6 g dieser Verbindung werden in einem Gemisch von 60 ml Ethanol / 60 ml Acetonitril mit 1 g Natriumborhydrid versetzt und 3 Stunden gerührt. Anschliessend werden 250 ml Eiswasser und 100 ml Essigester zugegeben, nach dem Zersetzen des Natriumborhydrids mit konzentrierter Essigsäure unter Rühren wird mit konzentrierter Ammoniaklösung alkalisch gemacht, die Essigesterphase wird abgetrennt, getrocknet und am Rotavapor eingeengt. Der ölige Rückstand wird in Äther gelöst, abgekühlt und das ausgefallene threo-5'-Benzyloxy-8'-(1-hydroxy-2-benzylisopropylamino-butyl)-2H-1,4-benzoxazin-3-(4H)-on (Fp. 89 - 92°C) abgesaugt.

4,8 g dieser Verbindung werden in 100 ml Methanol mit Palladiumkohle als Katalysator hydriert. Nach beendeter Aufnahme wird der Katalysator abgesaugt, die Mutterlauge wird am Rotavapor eingeengt, der ölige Rückstand wird in Aceton/Ethanol gelöst und mit der berechneten Menge Salzsäure angesäuert. Die Lösung wird mit Ether verdünnt und das ausgefallene threo-5'-Hydroxy-8'-(1-hydroxy-2-isopropylamino-butyl)-2H-1,4-benzoxazin-3-(4H)-on-hydrochlorid (Ausbeute 74% d. Th.) wird abgesaugt; nach Umfällen aus Methanol/Ether schmilzt es bei 202 - 205°C.

*Beispiel 2*

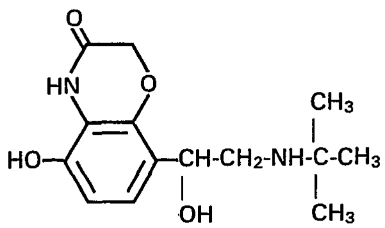

× HCl;

10 g 5'-Benzyloxy-8'-(1-oxo-2-brom-äthyl)-2H-1,4-benzoxazin-3-(4H)-on und 8,75 g Benzyl-tert.-butylamin werden in 100 ml Acetonitril 3 Stunden refluxiert. Nach dem Abkühlen werden die ausgefallenen Kristalle abgesaugt und mit 200 ml warmem Wasser gewaschen. Die Kristalle werden in Acetonitril mit ätherischer Salzsäure angesäuert; nach dem Verdünnen mit Essigester fällt 5'-Benzyloxy-8'-(1-oxo-2-benzyl-tert.-butylamino-äthyl)-2H-1,4-benzoxazin-3-(4H)-on hydrochlorid (Fp. 185 - 189°C) aus.

7 g dieser Verbindung werden bei 5 bar und 50°C in 100 ml Methanol unter Zusatz von Palladium-Kohle als Katalysator zu 5'-Hydroxy-8'-(1-oxo-2-tert.-butylamino-äthyl)-2H-1,4-benzoxazin-3-(4H)-on-hydrochlorid (Fp. 237 - 240°C) entbenzyliert.

Durch katalytische Hydrierung von 2,2 g dieser Verbindung in Methanol mit Platin erhält man 1,6 g 5'-Hydroxy-8'-(1-hydroxy-2-tert.-butylamino-äthyl)-2H-1,4-benzoxazin-3-(4H)-on hydrochlorid (Ausbeute: 72,5% d. Th.), welches bei 185 - 187°C schmilzt.

Entsprechend den angegebenen Beispielen werden synthetisiert:

*Tabelle I*

| Nr. | Strukturformel | Ausbeute % d. Th. | Salz mit | Schmelzpunkt °C |
|---|---|---|---|---|
| 1 | 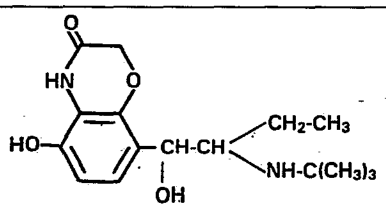 | 66 | Salzsäure × 2 Wasser | 230 (Zers.) |

*Tabelle I (Fortsetzung)*

| Nr. | Strukturformel | Ausbeute % d. Th. | Salz mit | Schmelzpunkt °C |
|---|---|---|---|---|
| 2 | 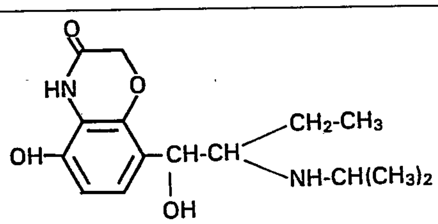 | 63 | Salzsäure × 1 Wasser | 163 - 165 |
| 3 | 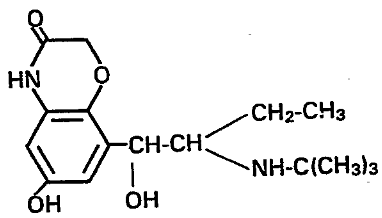 | 83 | Salzsäure | 259 - 261 |
| 4 | 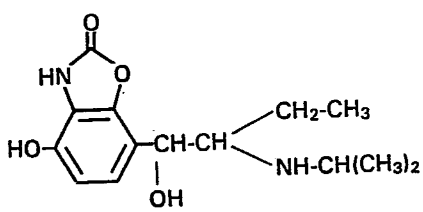 | 67 | Salzsäure | 230 - 232 |
| 5 | 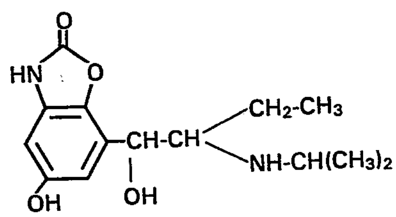 | 71 | Salzsäure × 1 Wasser | 256 - 259 |
| 6 | 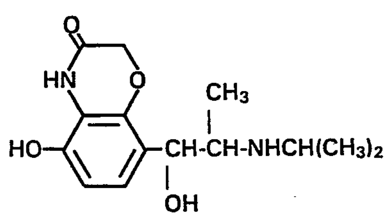 | 88 | HCl × 1/2 $H_2O$ | 244 |
| 7 | 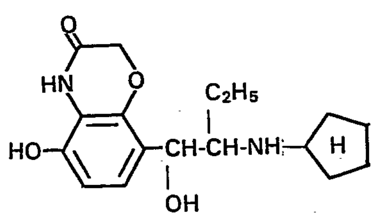 | 86 | HCl × 1/2 $H_2O$ | 243 - 245 |
| 8 | 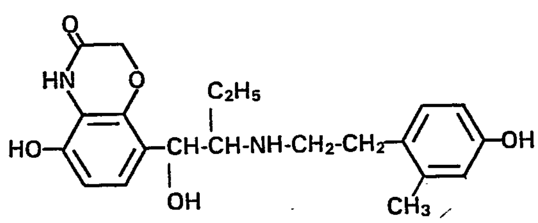 | 73,5 | HCl | 206 - 209 |

*Tabelle I (Fortsetzung)*

| Nr. | Strukturformel | Ausbeute % d. Th. | Salz mit | Schmelzpunkt °C |
|---|---|---|---|---|
| 9 | 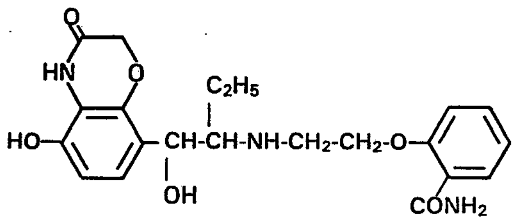 | 52 | HCl | 170 - 173 |
| 10 | 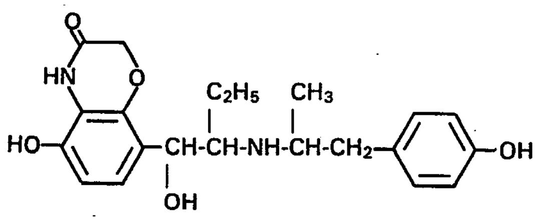 | 64 | $CH_3SO_3H$ × $H_2O$ | 197 - 201 |
| 11 | 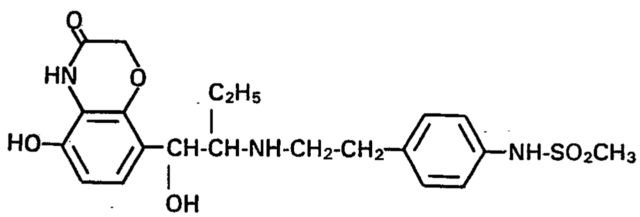 | 83 | $CH_3SO_3H$ | 187 - 190 |
| 12 | 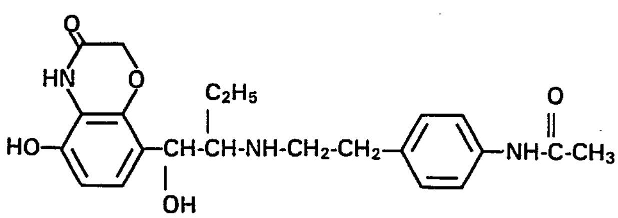 | 54 | HCl | 208 - 211 |
| 13 | 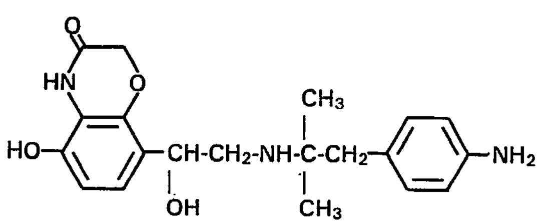 | 70 | HCl | 155 - 159 |
| 14 | 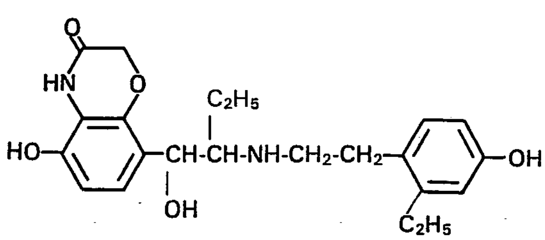 | 90 | HCl<br>$CH_3SO_3H$ × $H_2O$ | 234 - 236<br>92 - 94 |

*Tabelle I (Fortsetzung)*

| Nr. | Strukturformel | Ausbeute % d. Th. | Salz mit | Schmelzpunkt °C |
|---|---|---|---|---|
| 15 | 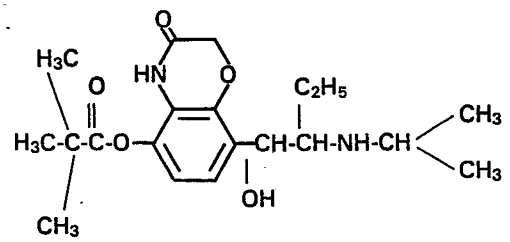 | | | |
| 16 | 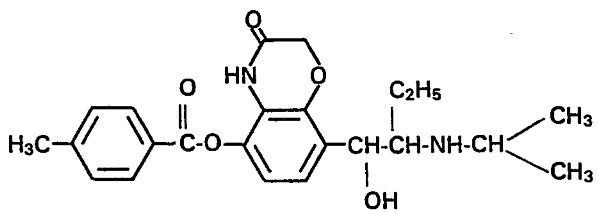 | | | |
| 17 | 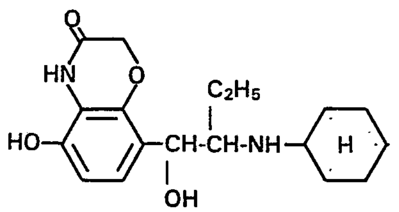 | 90 | HCl | 234 - 236 |
| | | | $CH_3SO_3H$ × $H_2O$ | 92 - 94 |

*Zu Verfahren 2*

*Beispiel 3*

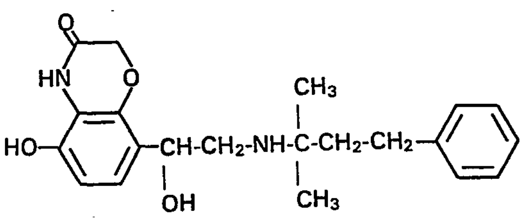
× HCl

5 g 5'-Benzyloxy-8'-(1-oxo-2-hydroxy-2-äthoxy--äthyl)-2H-1,4-benzoxazin-3-(4H)-on, 2,2 g 1,1-Dimethyl-3-phenylpropylamin und 50 ml Alkohol werden 3 Stunden auf 50 - 60°C erwärmt. Nach Abkühlen des Reaktionsgemisches wird die ausgefallene Schiffsche Base (Fp. 138 - 140°C) abgesaugt.

4,5 g dieser Verbindung werden in 100 ml Alkohol mit 1 g Natriumborhydrid versetzt und 2 Stunden bei Raumtemperatur gerührt. Nach Zugabe von 100 ml Wasser wird das ausgefallene 5'-Benzyloxy-8'-[1--hydroxy-2-(4-phenyl-2-methylbutylamino)-äthyl]--2H-1,4-benzoxazin-3-(4H)-on (Fp. 162 - 164°C) abgesaugt und mit ätherischer Salzsäure das Hydrochlorid hergestellt (Fp. 205 - 207°C). Durch katalytische Hydrierung dieser Verbindung in 50 ml Methanol unter Normalbedingungen mit Palladium--Kohle als Katalysator erhält man 2,7 g 5'-Hydroxy--8'-[1-Hydroxy-2-(4-phenyl-2-methylbutylamino)--äthyl]-2H-1,4-benzoxazin-3-(4H)-on-hydrochlorid (Fp. 159 - 161°C), Ausbeute: 90% d. Th.

*Beispiel 4*

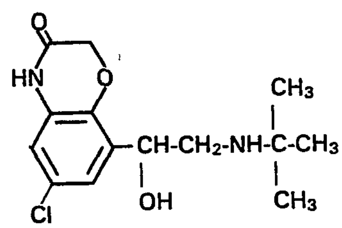
× HCl

5,8 g 6'-Chlor-8'-(1-oxo-2-hydroxy-2-äthoxy--äthyl)-2H-1,4-benzoxazin-3-(4H)-on, 1,5 g tert.--Butylamin, 60 ml Dioxan und 60 ml Alkohol werden 2 Stunden auf 50°C erwärmt. Anschliessend wird abgekühlt und die Lösung bei 10 - 20°C mit 2 g Natriumborhydrid versetzt. Die Lösung wird eine Stunde bei Raumtemperatur gerührt, dann auf 500 ml Eiswasser gegeben und mit 150 ml Essigester überschichtet. Nach Zersetzen des Natriumborhydrids unter Rühren mit konz. Essigsäure wird mit wässrigem Ammoniak alkalisch gestellt, die Essigesterphase abgetrennt, mit Natriumsulfat getrocknet und am Rotavapor eingeengt. Der ölige Rückstand wird in 15 ml Alkohol gelöst, mit ätherischer Salzsäure angesäuert und das ausgefallene 6'-Chlor-8'-[1-hydroxy--2-(tert.-butylamino)-äthyl]-2H-1,4-benzoxazin-3--(4H)-on-hydrochlorid (Ausbeute: 38% d. Th.) abgesaugt. Nach zweimaligem Umfällen aus Methanol unter Zusatz von Aktivkohle hat die Substanz einen Schmelzpunkt, der über 300°C liegt (Fp. der Base: 173 - 177°C).

In analoger Weise werden folgende Verbindungen hergestellt:

*Tabelle II*

| Nr. | Strukturformel | Ausbeute % d. Th. | Salz mit | Schmelzpunkt °C |
|---|---|---|---|---|
| 1 | 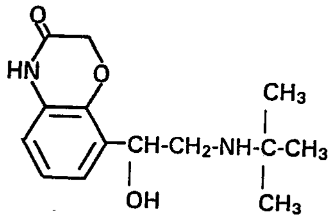 | 40 | Salzsäure | 252 - 255 |
| 2 | 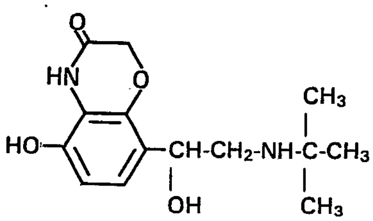 | 39 | Salzsäure | 185 - 187 |
| 3 | 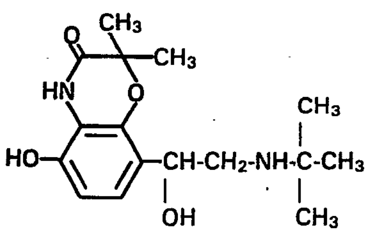 | 40 | Salzsäure × 1 Ethanol | 205 - 208 |
| 4 | 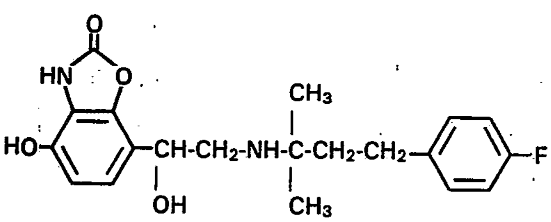 | 42 | Salzsäure × 1/2 Wasser | 155 - 160 |
| 5 | 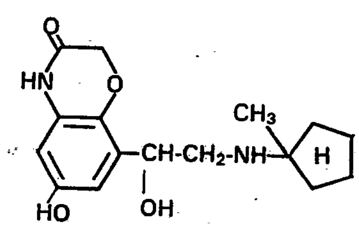 | 52 | Salzsäure | 226 - 229 |
| 6 | 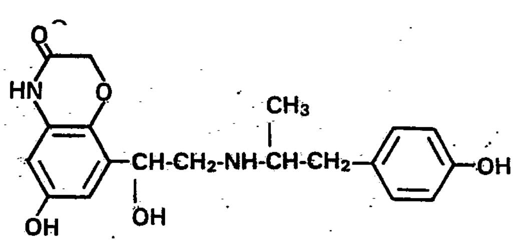 | 16 | Salzsäure | 206 - 209 |

*Tabelle II (Fortsetzung)*

| Nr. | Strukturformel | Ausbeute % d. Th. | Salz mit | Schmelzpunkt °C |
|---|---|---|---|---|
| 7 | 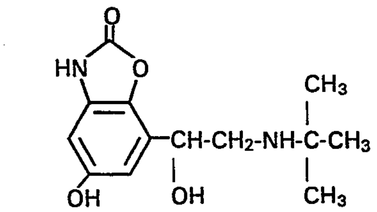 | 26 | Salzsäure × 1 Acetonitril | unscharf 195°C Zers. |
| 8 | 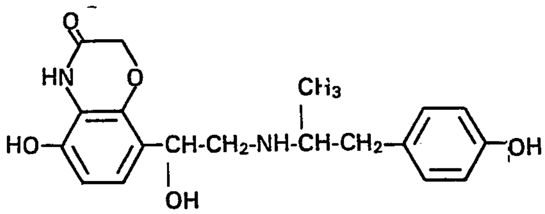 | 42 | HCl × $CH_3OH$ | 130 - 133 |
| 9 | 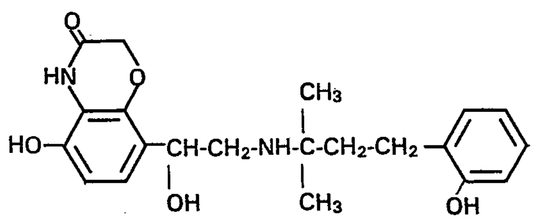 | 48 | HCOOH × $H_2O$ | 120 - 124 |
| 10 | 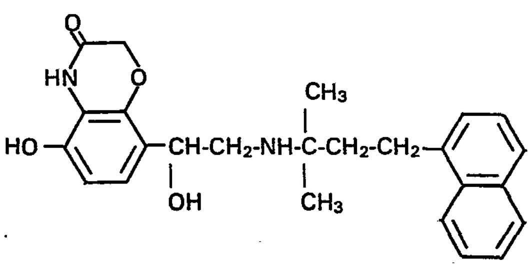 | 40 | $CH_3SO_3H$ | 192 - 195 |
| 11 | 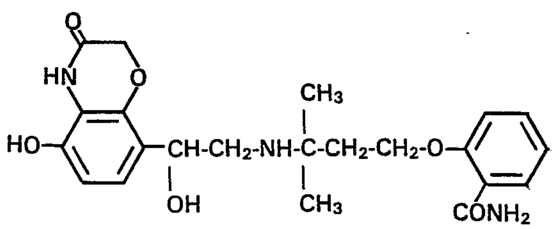 | 35 | HCl | 205 - 208 |

*Zu Verfahren 3*

*Beispiel 5*

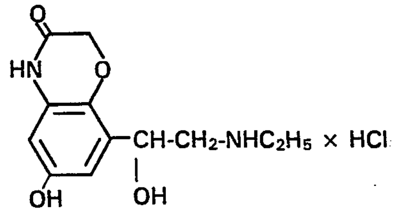

4,3 g 5'-Benzyloxy-8'-(1-hydroxy-2-benzyläthyl-amino-äthyl)-2H-1,4-benzoxazin-3-(4H)-on-hydrochlorid (Fp. 232 - 235°C) werden in 125 ml Methanol unter Zusatz von 0,5 g 5%iger Palladium-Kohle hydriert. Nach Aufnahme der berechneten Menge Wasserstoff wird der Katalysator abfiltriert und die Lösung unter vermindertem Druck abdestilliert. Durch Verreiben des Rückstandes mit Acetonitril erhält man 2,5 g 5'-Hydroxy-8'-(1-hydroxy-2-äthylamino-äthyl)-2H-1,4-benzoxazin-3-(4H)-on-hydrochlorid (Ausbeute: 86,7% d. Th.), welches nach dem Umfällen aus Methanol/Ether bei 240 - 242°C schmilzt.

*Beispiel 6*

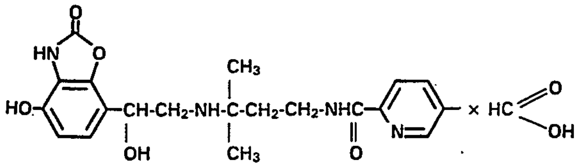

6,3 g 4'-Benzyloxy-7'-[1-hydroxy-2-(4-picolinsäure-amido-2-methyl-2-butylamino)-äthyl]-2-benzoxazolinon (Fp. 130 - 133°C) werden in 125 ml Methanol unter Zusatz von 1 g 5%iger Palladiumkohle hydriert. Nach beendeter Aufnahme wird der Katalysator abfiltriert und die klare Lösung am Rotavapor unter vermindertem Druck eingedampft. Der ölige Rückstand wird in 10 ml Alkohol gelöst und mit 0,58 g Ameisensäure versetzt. Nach 5 Stunden wird das ausgefallene 4'-Hydroxy-7'-[1-hydroxy-2-(4-picolinsäure-amido-2-methyl-2-butylamino)-2-benzoxazolinon]-formiat (Ausbeute: 78,5% d. Th., Fp. 166 - 168°C) abgesaugt.

Entsprechend den angegebenen Beispielen wurden synthetisiert:

*Tabelle III*

| Nr. | Strukturformel | Ausbeute % d. Th. | Salz mit | Schmelzpunkt °C |
|---|---|---|---|---|
| 1 | 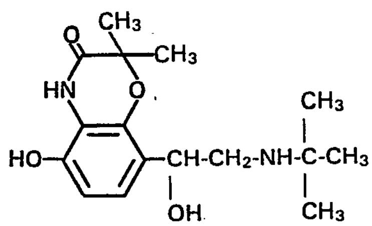 | 87 | Salzsäure × 1 Ethanol | 205 - 208 |
| 2 | 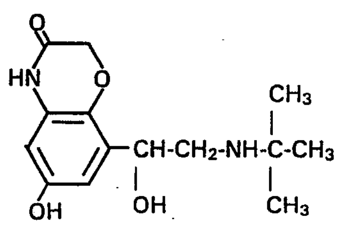 | 75 | Salzsäure × 1 Ethanol | 246 - 247 |
| 3 | 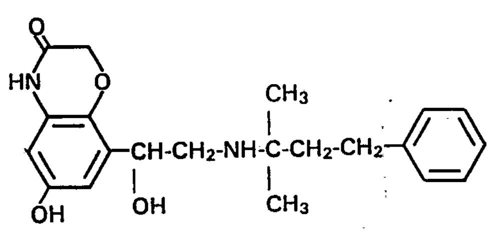 | 70 | Salzsäure × 1 Ethanol | 120 - 123 |
| 4 | 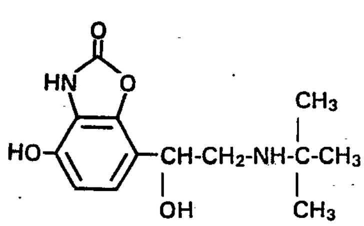 | 70 | Ameisensäure × 1 Wasser | 189 - 192 |

*Tabelle III (Fortsetzung)*

| Nr. | Strukturformel | Ausbeute % d. Th. | Salz mit | Schmelzpunkt °C |
|---|---|---|---|---|
| 5 | 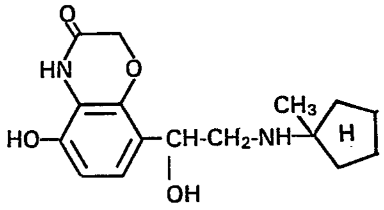 | 88 | Salzsäure | 226 - 229 |
| 6 | 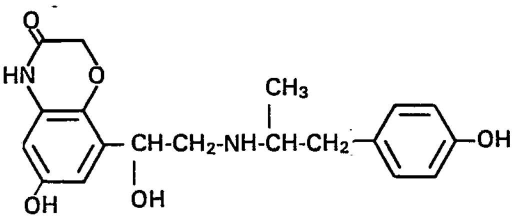 | 78,5 | Salzsäure | 206 - 209 |
| 7 | 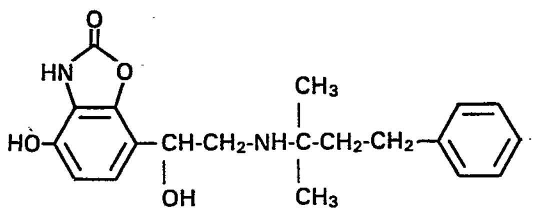 | 75 | Salzsäure | 174 - 175 |
| 8 | 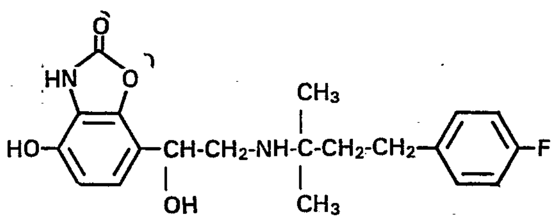 | 90 | Salzsäure × 1/2 Wasser | 155 - 160 |
| 9 | 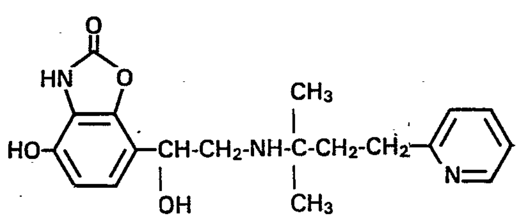 | 75 | 1/2 Fumarsäure | 175 - 178 (170 - 173 Base) |
| 10 | 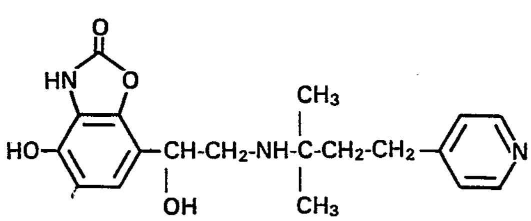 | 60 | Salzsäure | 143 - 146 |

*Tabelle III (Fortsetzung)*

| Nr. | Strukturformel | Ausbeute % d. Th. | Salz mit | Schmelzpunkt °C |
|---|---|---|---|---|
| 11 | 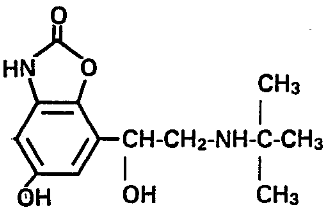 | 76 | Salzsäure × 1 Acetonitril | unscharf 195 Zers. |
| 12 | 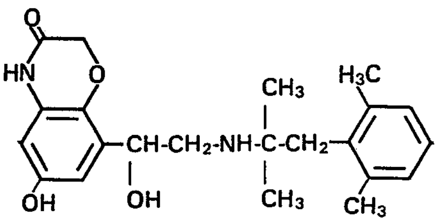 | 91 | $CH_3SO_3H$ × 1 $H_2O$ | 252 - 254 |
| 13 | 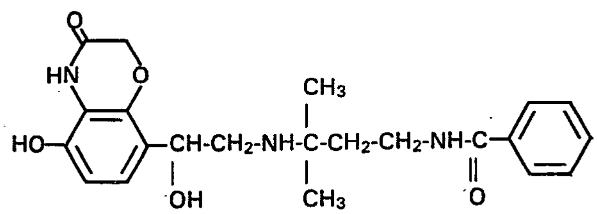 | 71 | $CH_3SO_3H$ × 1/2 $H_2O$ | 178 - 180 |
| 14 | 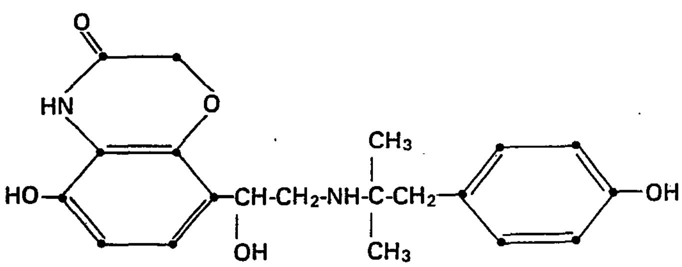 | 72 | HCl × 1,5 $H_2O$ | 159 - 162 |
| 15 | 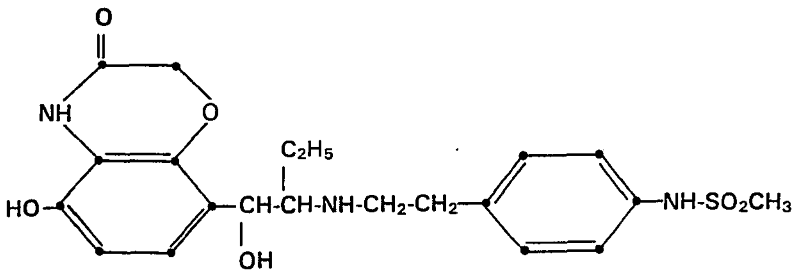 | | | |
| 16 | 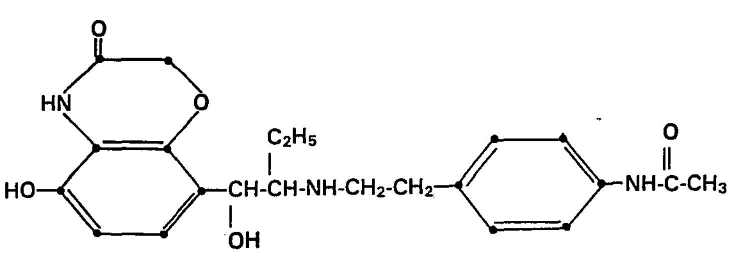 | | | |

*Tabelle III (Fortsetzung)*

| Nr. | Strukturformel | Ausbeute % d. Th. | Salz mit | Schmelzpunkt °C |
|---|---|---|---|---|
| 17 | 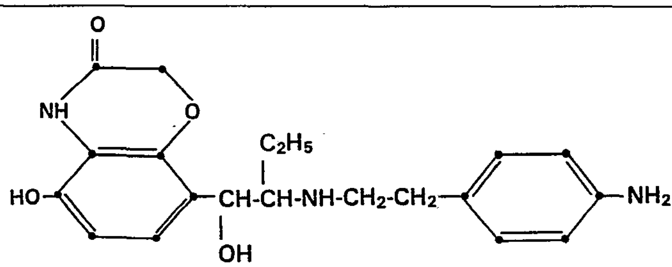 | | | |
| 18 | 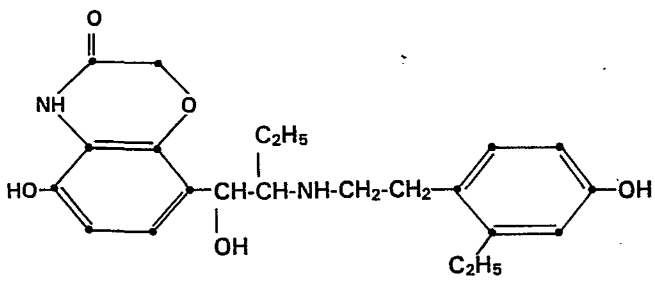 | | | |
| 19 | 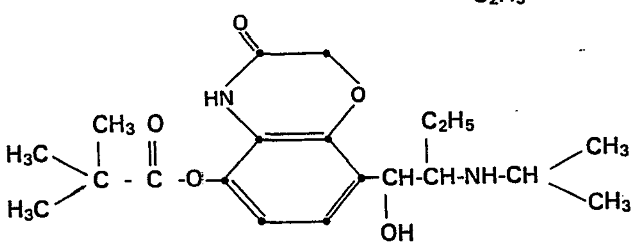 | | | |

Nachstehend sind Zwischenprodukte der Formel V angegeben, die entsprechend dem obigen Schema erhalten werden können.

Die Verbindungen können auch selbst als Arzneistoffe verwendet werden, da sie ähnliche pharmakologische Eigenschaften wie die Endprodukte der Formel I aufweisen.

| Formel | Salz mit | Schmelzpunkt °C |
|---|---|---|
| 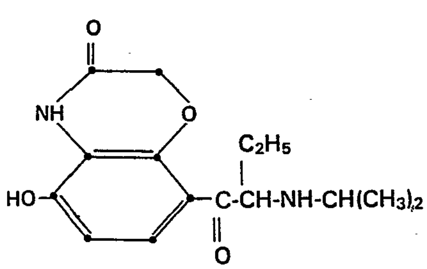 | HCl × $2H_2O$ | 240 - 242 |
| 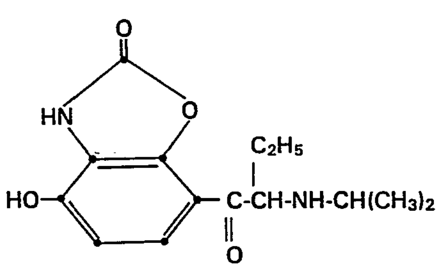 | HCl | 218 - 222 |

| Formel | Salz mit | Schmelzpunkt °C |
|---|---|---|
| 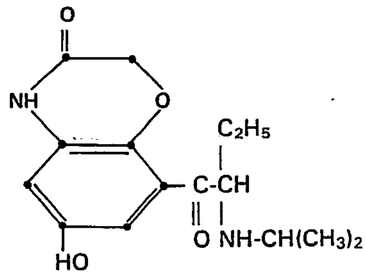 | HCl | 250 - 254 |
| 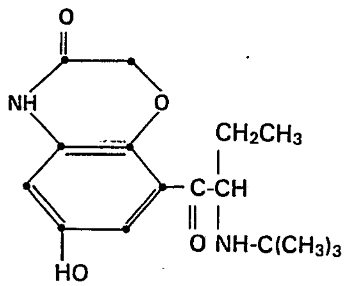 | HCl | 250 - 253 |
| 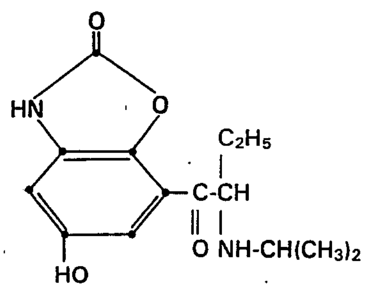 | HCl | 217 - 223 |
| 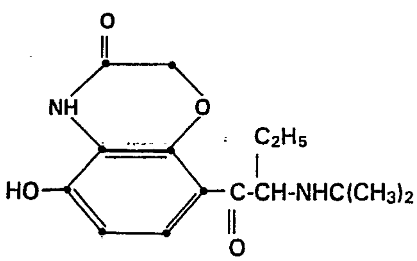 | HCl | 156 - 161 |
| 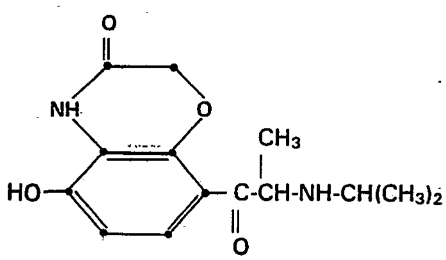 | HCl | 243 - 247 |
| 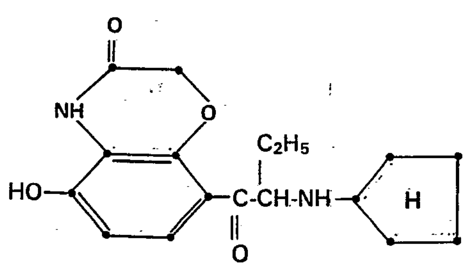 | HCl | 254 - 258 |

| Formel | Salz mit | Schmelzpunkt °C |
|---|---|---|
| 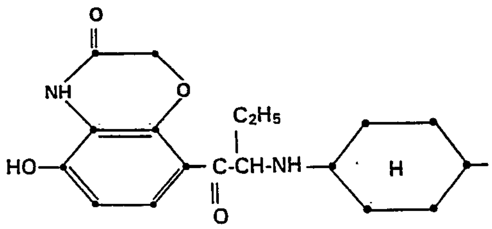 | HCl | 250 |

**Patentansprüche**

1. Verbindungen der allgemeinen Formel

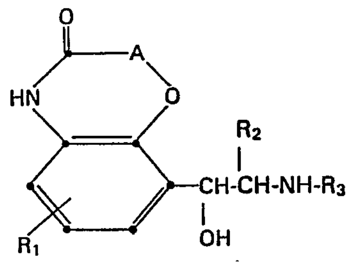 (I)

in der

A eine Einfachbindung, die Gruppe $=C<^{R_5}_{R_4}$

oder die Gruppe $-CH_2-CH_2$,
$R_1$ OH, O-($C_1$-$C_6$-Alkanoyl), O-Benzoyl, O-p-Methylbenzoyl oder Chlor
$R_2$ Wasserstoff, Methyl oder Ethyl,

$R_3$ 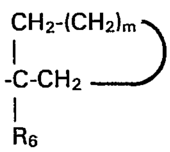 oder 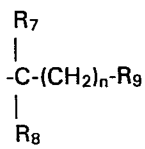

(II) (III)

m 2, 3 oder 4,
n 1, 2 oder 3,
$R_4$ Wasserstoff, $C_1$-$C_4$-Alkyl
$R_5$ Wasserstoff, $C_1$-$C_4$-Alkyl und ausserdem, falls $R_4$ Wasserstoff ist, Phenyl,
$R_6$ Wasserstoff oder Methyl,
$R_7$ Wasserstoff oder Methyl,
$R_8$ Wasserstoff oder Methyl,
$R_9$ Wasserstoff, Ar, OAr, NH-CO-Ar

Ar 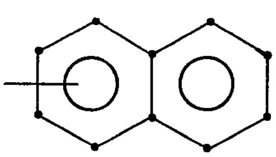 , 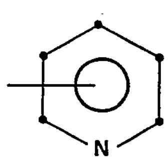 oder

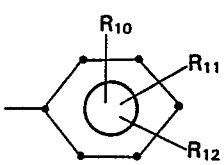 (IV)

$R_{10}$, $R_{11}$, $R_{12}$ (die gleich oder verschieden sein können), Wasserstoff, Hydroxy, Methyl, Methoxy, Halogen, Methylendioxy, NH-$R_{13}$, $CONH_2$,
$R_{13}$ Wasserstoff, $C_1$-$C_6$-Alkanoyl oder Benzoyl oder $C_1$-$C_4$-Alkylsulfonyl bedeutet,
in Form von Racematen, Enantiomeren und ggf. diastereomeren Antipodenpaaren, jeweils als freie Basen oder als Säureadditionssalze.

2. Die Verbindung 5'-Hydroxy-8'-(1-hydroxy-2--isopropylamino-butyl)-2H-1,4-benzoxazin-3-(4H)--on und ihre Salze.

3. Pharmazeutische Zubereitungen, gekennzeichnet durch einen Gehalt an einer Verbindung nach Anspruch 1 oder 2 neben üblichen Hilfs- und Trägerstoffen.

4. Verfahren zur Herstellung von Verbindungen der allgemeinen Formel

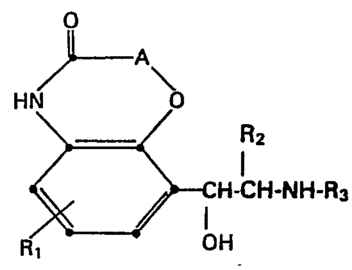 (I),

in der

A eine Einfachbindung, die Gruppe $=C<^{R_5}_{R_4}$

oder die Gruppe $-CH_2-CH_2$,
$R_1$ OH, O-($C_1$-$C_6$-Alkanoyl), O-Benzoyl, O-p-Methylbenzoyl oder Chlor
$R_2$ Wasserstoff, Methyl oder Ethyl,

$R_3$ 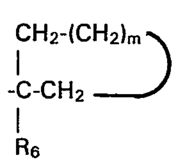 oder 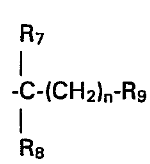

(II) (III)

m 2, 3 oder 4,
n 1, 2 oder 3,
$R_4$ Wasserstoff, $C_1$-$C_4$-Alkyl
$R_5$ Wasserstoff, $C_1$-$C_4$-Alkyl und ausserdem, falls $R_4$ Wasserstoff ist, Phenyl,
$R_6$ Wasserstoff oder Methyl,
$R_7$ Wasserstoff oder Methyl,

$R_8$ Wasserstoff oder Methyl,
$R_9$ Wasserstoff, Ar, OAr, NH-CO-Ar

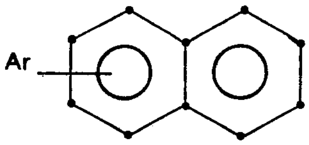 , 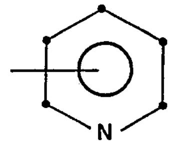 oder

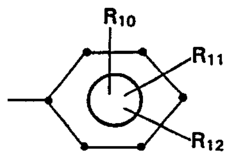 (IV)

$R_{10}$, $R_{11}$, $R_{12}$ (die gleich oder verschieden sein können), Wasserstoff, Hydroxy, Methyl, Methoxy, Halogen, Methylendioxy, NH-$R_{13}$, $CONH_2$,
$R_{13}$ Wasserstoff, $C_1$-$C_6$-Alkanoyl oder Benzoyl oder $C_1$-$C_4$-Alkylsulfonyl bedeutet, in Form von Racematen, Enantiomeren und gegebenenfalls diastereomeren Antipodenpaaren, jeweils als freie Basen oder als Säureadditionssalze, dadurch gekennzeichnet, dass man

a) eine Verbindung der Formel

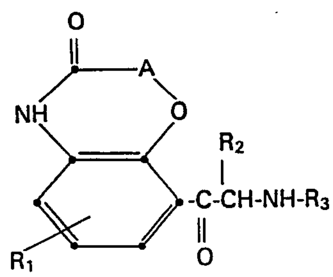 (V)

in der A, $R_1$, $R_2$ und $R_3$ die obige Bedeutung haben, wobei phenolische OH-Gruppen auch durch hydrogenolytisch abspaltbare Schutzgruppen geschützt vorliegen können, reduziert und ggf. anschliessend noch vorhandene Schutzgruppen abspaltet
oder dass man

b) ein Phenylglyoxal oder Halbacetal der Formel

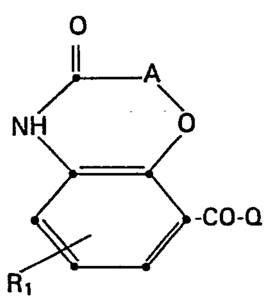 (XII),

in der $R_1$ und A die obige Bedeutung haben, wobei jedoch phenolische OH-Gruppen durch hydrogenolytisch abspaltbare Schutzgruppen geschützt vorliegen können, und Q für -CHO oder -CH(OH)-O-Niederalkyl steht, mit einem Amin der Formel

$$H_2N\text{-}R_3 \quad (XIII),$$

worin $R_3$ die obige Bedeutung hat, wobei etwa enthaltene OH-Gruppen durch hydrogenolytisch abspaltbare Schutzgruppen geschützt vorliegen können, unter den Bedingungen der reduktiven Aminierung umsetzt und ggf. anschliessend noch vorhandene Schutzgruppen abspaltet oder dass man

c) aus einer Verbindung der Formel

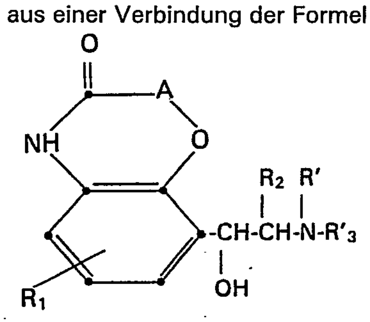 (XVI),

in der A und $R_2$ die obige Bedeutung haben und $R'_1$ für $R_1$ oder eine durch eine hydrogenolytisch abspaltbare Schutzgruppe geschützte OH-Gruppe, $R'_3$ für $R_3$ steht, wobei jedoch eine in $R_3$ etwa enthaltene OH-Gruppe durch eine hydrogenolytisch abspaltbare Schutzgruppe geschützt sein kann, und R' für Wasserstoff oder eine hydrogenolytisch abspaltbare Schutzgruppe steht, und wobei mindestens eine abzuspaltende Schutzgruppe in der Verbindung der Formel XVI vorhanden ist, die Schutzgruppe nach üblichen Methoden abspaltet und dass man gewünschtenfalls die nach a) bis c) erhaltenen Verbindungen nach üblichen Methoden in die Enantiomeren, ggf. auch in die diastereomeren Antipodenpaare auftrennt, zunächst erhaltene Basen in Säureadditionssalze, zunächst erhaltene Säureadditionssalze in Basen oder Salze mit anderen Säuren überführt.

5. Verbindungen der Formel

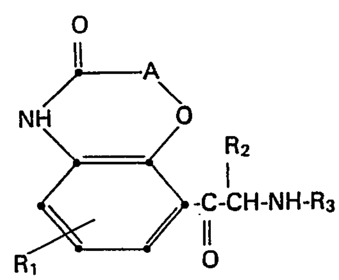 (V),

in der A, $R_1$, $R_2$ und $R_3$ die im Anspruch 1 angegebene Bedeutung haben.

6. Mittel zur Verbesserung der Fleischbildung und der Futterausnutzung bei fleischproduzierenden Tieren, gekennzeichnet durch einen Gehalt an einer Verbindung nach Anspruch 1.

7. Verwendung von Verbindungen nach Anspruch 1 zur Verbesserung der Fleischbildung und der Futterausnutzung bei fleischproduzierenden Tieren.

8. Verwendung von Verbindungen nach Anspruch 1 zur Verbesserung der Fleischbildung und Futterausnutzung bei Geflügel, Rindvieh, Schweinen und Schafen.

**Claims**

1. Compounds of general formula

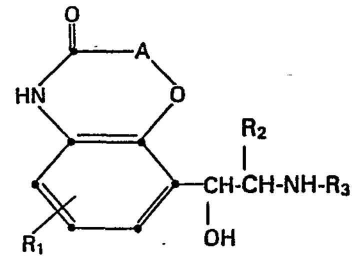

(I)

wherein

A represents a single bond, the group $=C\genfrac{}{}{0pt}{}{R_5}{R_4}$

or the group $-CH_2-CH_2$,
$R_1$ represents OH, O-($C_1$-$C_6$-alkanoyl), O-benzoyl, O-p-methylbenzoyl or chlorine,
$R_2$ represents hydrogen, methyl or ethyl,

$R_3$ represents 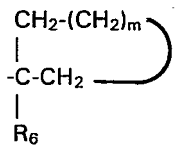 or 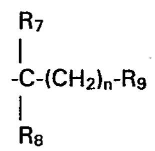

(II) (III)

m represents 2, 3 or 4
n represents 1, 2 or 3,
$R_4$ represents hydrogen or $C_{1-4}$ alkyl,
$R_5$ represents hydrogen, $C_{1-4}$ alkyl and also phenyl, if $R_4$ is hydrogen,
$R_6$ represents hydrogen or methyl,
$R_7$ represents hydrogen or methyl,
$R_8$ represents hydrogen or methyl,
$R_9$ represents hydrogen, Ar, OAr, NH-CO-Ar
Ar represents

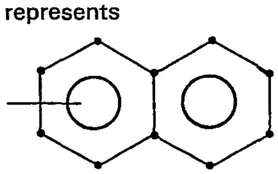 , 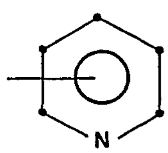 or

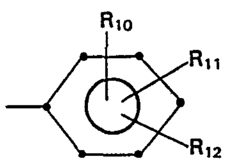

(IV)

$R_{10}$, $R_{11}$ and $R_{12}$ (which may be identical or different) represent hydrogen, hydroxy, methyl, methoxy, halogen, methylendioxy, NH-$R_{13}$ or $CONH_2$,
$R_{13}$ represents hydrogen, $C_{1-6}$ alkanoyl or benzoyl or $C_{1-4}$ alkylsulphonyl,
in the form of racemates, enantiomers and optionally diastereomeric pairs of enantiomers, all in the form of free bases or in the form of acid addition salts.

2. The compound 5'-hydroxy-8'-(1-hydroxy-2-isopropylaminobutyl)-2H-1,4-benzoxazin-3-(4H)-one and the salts thereof.

3. Pharmaceutical preparations, characterised in that they contain a compound as claimed in claim 1 or 2 together with conventional excipients and carriers.

4. Process for preparing compounds of general formula

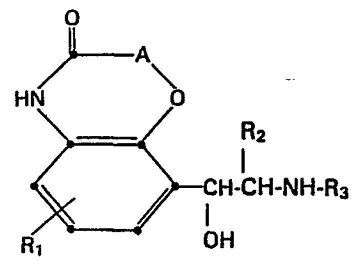

(I)

wherein

A represents a single bond, the group $=C\genfrac{}{}{0pt}{}{R_5}{R_4}$

or the group $-CH_2-CH_2$,
$R_1$ represents OH, O-($C_1$-$C_6$-alkanoyl), O-benzoyl, O-p-methylbenzoyl or chlorine,
$R_2$ represents hydrogen, methyl or ethyl,

$R_3$ represents 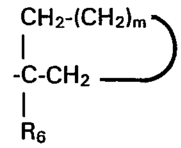 or 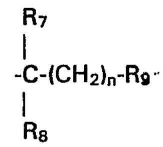

(II) (III)

m represents 2, 3 or 4
n represents 1, 2 or 3,
$R_4$ represents hydrogen or $C_{1-4}$ alkyl,
$R_5$ represents hydrogen, $C_{1-4}$ alkyl and also phenyl, if $R_4$ is hydrogen,
$R_6$ represents hydrogen or methyl,
$R_7$ represents hydrogen or methyl,
$R_8$ represents hydrogen or methyl,
$R_9$ represents hydrogen, Ar, OAr, NH-CO-Ar
Ar represents

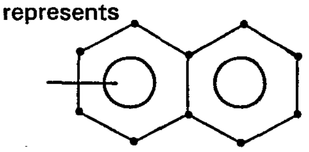 , 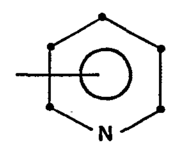 or

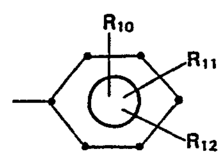

(IV)

$R_{10}$, $R_{11}$ and $R_{12}$ (which may be identical or different) represent hydrogen, hydroxy, methyl, methoxy, halogen, methylendioxy, NH-$R_{13}$ or $CONH_2$,
$R_{13}$ represents hydrogen, $C_{1-6}$ alkanoyl or benzoyl or $C_{1-4}$ alkylsulphonyl,
in the form of racemates, enantiomers and optionally diastereomeric pairs of enantiomers, all in the form of free bases or in the form of acid addition salts, characterised in that

a) a compound of formula

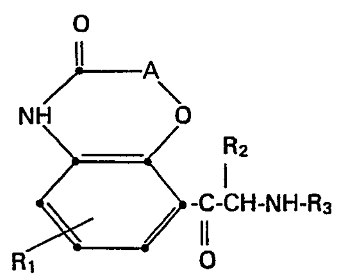

(V)

wherein A, $R_1$, $R_2$ and $R_3$ are as hereinbefore defined, and phenolic OH groups may also be present, protected by hydrogenolytically removable protecting groups, is reduced and subsequently any protecting groups still present are split off
or

b) a phenylglyoxal or hemiacetal of formula

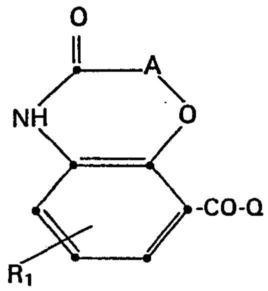

(XII)

wherein $R_1$ and A are as hereinbefore defined, but phenolic OH groups may be present, protected by hydrogenolytically removable protecting groups, and Q represents -CHO or -CH(OH)-O-lower alkyl, is reacted with an amine of formula

$$H_2N\text{-}R_3 \qquad (XIII)$$

wherein $R_3$ is as hereinbefore defined, and any OH groups contained may be present protected by hydrogenolytically removable protecting groups, under the conditions of reductive amination and subsequently any protecting groups still present are split off or

c) the protecting group is split off, by conventional methods, from a compound of formula

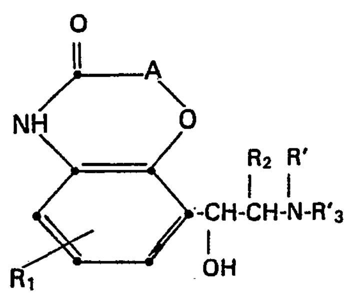

(XVI)

wherein A and $R_2$ are as hereinbefore defined and $R'_1$ represents $R_1$ or an OH group protected by a hydrogenolytically removable protecting group, $R'_3$ represents $R_3$, but any OH group contained in $R_3$ may be protected by a hydrogenolytically removable protecting group, and R' represents hydrogen or a hydrogenolytically removable protecting group, and at least one protecting group which is to be split off is present in the compound of formula XVI
and if desired the compounds obtained according to a) to c) are resolved by conventional methods into their enantiomers, optionally also into the diastereomeric pairs of enantiomers, any bases obtained initially are converted into acid addition salts and any acid addition salts obtained initially are converted into bases or salts with other acids.

5. Compounds of formula

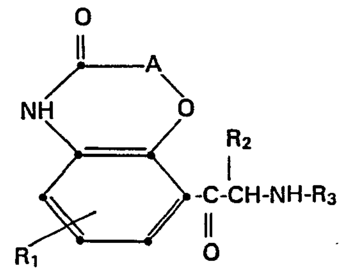

(V)

wherein
A, $R_1$, $R_2$ and $R_3$ have the meanings given in claim 1.

6. Agent for improving meat formation and the utilisation of fodder in meat-producing animals, characterised in that it contains a compound as claimed in claim 1.

7. Use of compounds as claimed in claim 1 for improving meat formation and the utilisation of fodder in meat-producing animals.

8. Use of compounds as claimed in claim 1 for improving meat formation and the utilisation of fodder in poultry, cattle, pigs and sheep.

**Revendications**

1. Composés de formule générale:

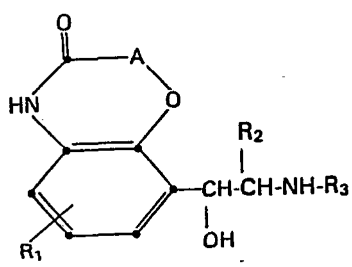

(I)

dans laquelle:

A représente une liaison simple, le groupe $=C\langle^{R_5}_{R_4}$
ou le groupe $-CH_2-CH_2$,
$R_1$ représente OH, O-(alcanoyle en $C_1$-$C_6$), O-benzoyle, O-p-méthylbenzoyle ou chlore,
$R_2$ représente hydrogène, méthyle ou éthyle,

$R_3$ représente 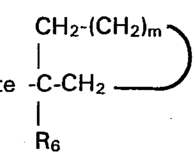 ou 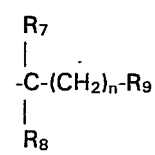

(II) (III)

m représente 2, 3 ou 4,
n représente 1, 2 ou 3,
$R_4$ représente hydrogène, alcoyle en $C_1$-$C_4$,
$R_5$ représente hydrogène, alcoyle en $C_1$-$C_4$ et en outre, lorsque $R_4$ est hydrogène, phényle,
$R_6$ représente hydrogène ou méthyle,
$R_7$ représente hydrogène ou méthyle,
$R_8$ représente hydrogène ou méthyle,
$R_9$ représente hydrogène, Ar, OAr, NH-CO-Ar,
Ar représente

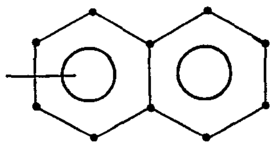, 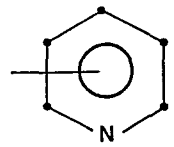 ou

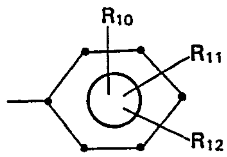

(IV)

$R_{10}$, $R_{11}$, $R_{12}$ (qui peuvent être identiques ou différents), représentent hydrogène, hydroxy, méthyle, méthoxy, halogène, méthylènedioxy, NH-$R_{13}$, $CONH_2$,
$R_{13}$ représente hydrogène, alcanoyle en $C_1$-$C_6$ ou benzoyle ou alcoylsulfonyle en $C_1$-$C_4$,
sous forme de racémates, d'énantiomères et éventuellement de paires d'antipodes diastéréoisomères, dans chaque cas sous forme de bases libres ou sous forme de sels d'addition d'acides.

2. Le composé 5'-hydroxy-8'-(1-hydroxy-2-isopropylaminobutyl)-2H-1,4-benzoxazine-3-(4H)-one et ses sels.

3. Préparations pharmaceutiques, caractérisées par une teneur en un composé selon la revendication 1 ou 2, conjointement à des adjuvants et excipients usuels.

4. Procédé pour la préparation de composé de formule générale:

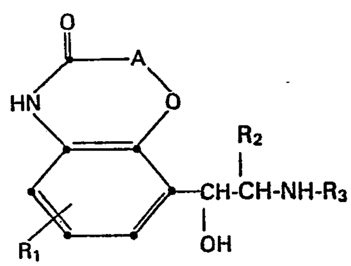

(I),

dans laquelle:

A représente une liaison simple, le groupe $=C<^{R_5}_{R_4}$

ou le groupe -$CH_2$-$CH_2$,
$R_1$ représente OH, O-(alcanoyle en $C_1$-$C_6$), O-benzoyle, O-p-méthylbenzoyle ou chlore,
$R_2$ représente hydrogène, méthyle ou éthyle

$R_3$ représente 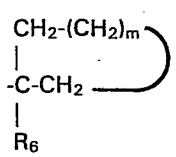 ou 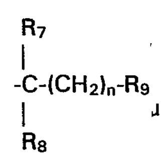

(II) (III)

m représente 2, 3 ou 4,
n représente 1, 2 ou 3,
$R_4$ représente hydrogène, alcoyle en $C_1$-$C_4$,
$R_5$ représente hydrogène, alcoyle en $C_1$-$C_4$, et en outre, lorsque $R_4$ est hydrogène, phényle,
$R_6$ représente hydrogène ou méthyle,
$R_7$ représente hydrogène ou méthyle,
$R_8$ représente hydrogène ou méthyle,
$R_9$ représente hydrogène, Ar, OAr, NH-CO-Ar,
Ar représente

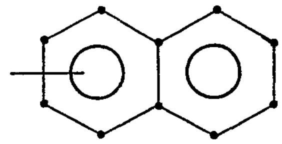, 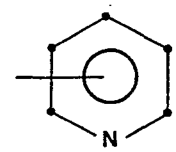 ou

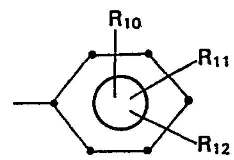

(IV)

$R_{10}$, $R_{11}$, $R_{12}$ (qui peuvent être identiques ou différents), représentent hydrogène, hydroxy, méthyle, méthoxy, halogène, méthylènedioxy, NH-$R_{13}$, $CONH_2$,
$R_{13}$ représente hydrogène, alcanoyle en $C_1$-$C_6$ ou benzoyle ou alcoylsulfonyle en $C_1$-$C_4$, sous forme de racémates, d'énantiomères et éventuellement de paires d'antipodes diastéréoisomères, dans chaque cas sous forme de bases libres ou sous forme de sels d'addition d'acides, caractérisé en ce que:

a) on réduit un composé de formule:

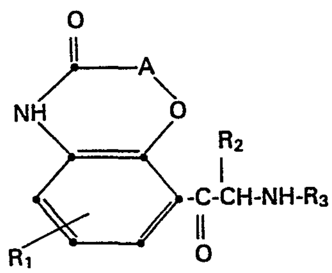

(V)

dans laquelle A, $R_1$, $R_2$ et $R_3$ ont la signification ci-dessus, des groupes OH phénoliques pouvant se présenter également protégés par des groupes protecteurs clivables hydrogénolytiquement et en ce qu'éventuellement ensuite, on clive des groupes protecteurs encore présents, ou en ce que

b) on fait réagir un phénylglyoxal ou semi-acétal de formule:

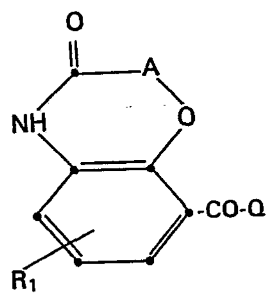

(XII),

dans laquelle $R_1$ et A ont la signification ci-dessus, des groupes OH phénoliques pouvant cependant se présenter protégés par des groupes protecteurs clivables hydrogénolytiquement et Q remplace -CHO ou -CH(OH)-O-alcoyle inférieur, avec une amine de formule:

$$H_2N\text{-}R_3 \qquad (XIII),$$

dans laquelle $R_3$ a la signification ci-dessus, d'éventuels groupes OH contenus dans cette formule pouvant se présenter protégés par des groupes protecteurs clivables hydrogénolytiquement, dans les conditions de l'amination réductrice et éventuellement ensuite on clive des groupes protecteurs encore présents, ou en ce que

c) on clive selon des méthodes usuelles les groupes protecteurs à partir d'un composé de formule:

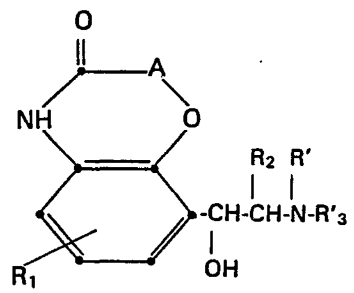

(XVI),

dans laquelle A et $R_2$ ont la signification ci-dessus et $R'_1$ remplace $R_1$ ou un groupe OH protégé par un groupe protecteur clivable hydrogénolytiquement, $R'_3$ remplace $R_3$, un groupe OH éventuellement contenu dans $R_3$ pouvant cependant être protégé par un groupe protecteur clivable hydrogénolytiquement, et R' remplace l'hydrogène ou un groupe protecteur clivable hydrogénolytiquement, et au moins l'un des groupes protecteurs à cliver étant présent dans le composé de formule XVI,
et en ce que, si on le désire, on sépare les composés obtenus selon a) à c), selon des méthodes usuelles, en les énantiomères, éventuellement aussi en les paires d'antipodes diastéréoisomères, transforme les bases d'abord obtenues en sels d'addition d'acides, les sels d'addition d'acides d'abord obtenus en bases ou sels avec d'autres acides.

5. Composés de formule:

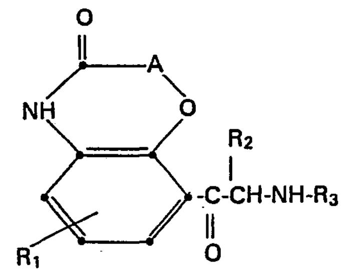

(V),

dans laquelle A, $R_1$, $R_2$ et $R_3$ ont la signification indiquée dans la revendication 1.

6. Agent pour améliorer la formation de viande et la mise à profit de la nourriture chez des animaux produisant de la viande, caractérisé par une teneur en un composé selon la revendication 1.

7. Utilisation de composés selon la revendication 1 pour améliorer la formation de viande et la mise à profit de la nourriture chez des animaux produisant de la viande.

8. Utilisation de composés selon la revendication 1 pour améliorer la formation de viande et la mise à profit de la nourriture chez des volailles, bovins, porcs et moutons.